# EUROPÄISCHE PATENTANMELDUNG

(11) **EP 4 800 708 A1**
(43) Veröffentlichungstag der Anmeldung: **02.09.2026**
(21) Anmeldenummer: 25160512.7
(22) Anmeldetag: 27.02.2025
(51) Int. Cl.: G16H 50/30, G16H 50/70

(54) **COMPUTERIMPLEMENTIERTES VERFAHREN ZUR DURCHFÜHRUNG EINER LEISTUNGSANALYSE EINES BENUTZERS**

(71) Anmelder: Research Industrial Systems Engineering (RISE) Forschungs-, Entwicklungs- und Grossprojektberatung GmbH, 2320 Schwechat (AT)
(72) Erfinder: HOELBLING , Dominik, 2320 Schwechat (AT); GRECHENIG , Thomas, 2320 Schwechat (AT); SCHOLZ , Sebastian, 2320 Schwechat (AT); MITTER, Benedikt, 2320 Schwechat (AT)
(74) Vertreter: Schwarz & Partner Patentanwälte GmbH

(57) **Zusammenfassung**

Die Erfindung betrifft ein computerimplementiertes Verfahren zur Durchführung einer Leistungsanalyse eines ersten Benutzers (B1), umfassend die Schritte:
- Bereitstellen einer Datenbank (1) mit einer Vielzahl von Datensätzen (DSi), wobei jeder Datensatz (DSi) eine Vielzahl von personenbezogenen Daten (pD) und zumindest einen ersten Leistungsmesswert (LM) umfasst,
- Empfangen von personenbezogenen Daten (pDB1-1, pDB1-2) des ersten Benutzers (B1) und eines gemessenen Leistungsmesswertes (LMB1-1) des ersten Benutzers (B1),
- Bestimmen von zumindest zwei unterschiedlichen Untergruppen (UG1, UG2, UG3) der Datenbank (1),
- für die genannten Untergruppen (UG1, UG2, UG3), Ermitteln zumindest einer statistischen Eigenschaft,
- Heranziehen jener Untergruppe (UG1, UG2, UG3), die der gemäß der zumindest einen ermittelten statistischen Eigenschaft am repräsentativsten für den ersten Benutzer (B1) ist,
- Durchführen der Leistungsanalyse des ersten Benutzers (B1) durch Ermitteln eines Leistungsergebnisses des Leistungsmesswerts (LMB1-1) des ersten Benutzers (B1) innerhalb der herangezogenen Untergruppe (UG1, UG2, UG3).

## Beschreibung

Die hierin beschriebene Erfindung betrifft ein computerimplementiertes Verfahren zur Durchführung einer Leistungsanalyse eines Benutzers.

Leistungsanalysen spielen in zahlreichen Bereichen eine zentrale Rolle, insbesondere in der Rehabilitation, in der Physiotherapie und im Leistungssport. In der Physiotherapie ermöglicht eine objektive Leistungsbewertung dem Therapeuten, den Zustand des Patienten zu testen und Fortschritt zu verfolgen, Therapiepläne individuell anzupassen und eine evidenzbasierte Rehabilitation zu gewährleisten. Für Leistungssportler ist es entscheidend, ihre körperliche Verfassung und Trainingsfortschritte kontinuierlich zu überwachen, um die Effektivität ihres Trainingsplans zu maximieren und das Verletzungsrisiko zu minimieren. Die Verfügbarkeit genauer und effizienter Analysemethoden ist somit von großer Bedeutung, um fundierte Entscheidungen für das Training oder die Therapie zu treffen.

Bestehende Leistungsanalysen werden traditionell durch manuelle Verfahren oder einfache elektronische Systeme durchgeführt. Im Leistungssport werden häufig Messgeräte wie Pulsmesser, Schrittzähler oder Kraftsensoren eingesetzt, um spezifische Parameter wie Herzfrequenz, Schrittfrequenz oder Kraftentwicklung zu erfassen. Solche Geräte liefern jedoch häufig nur isolierte Daten, die eine ganzheitliche Analyse erschweren. Zudem erfordern sie häufig die manuelle Interpretation der Ergebnisse durch Trainer oder Athleten, was zeitaufwendig und fehleranfällig sein kann.

Ein weiteres Problem besteht darin, dass Therapeuten eine Vielzahl an Messsystemen kennen müssen, um deren Ergebnisse sinnvoll interpretieren zu können. Dies erfordert nicht nur umfassendes Fachwissen, sondern auch viel Erfahrung, insbesondere wenn die Daten im Kontext einer bestimmten Erkrankung betrachtet werden. Zudem hat sich gezeigt, dass Therapeuten und Trainer bei der Analyse von Daten nicht immer frei von Vorurteilen sind. Dies kann dazu führen, dass Leistungsanalysen subjektiv beeinflusst werden und somit nicht in jedem Fall reproduzierbar sind.

In der Physiotherapie und Rehabilitation kommen zunehmend digitale Lösungen wie tragbare Sensoren oder Applikationen zum Einsatz, die Bewegungsmuster und biomechanische Parameter aufzeichnen. Diese Systeme sind jedoch häufig auf spezielle Übungen oder Geräte beschränkt und bieten nur begrenzte Möglichkeiten zur Integration verschiedener Datenquellen. Zudem fehlt es oft an einer Lösung, die es erlaubt, die gesammelten Informationen in einen aussagekräftigen Kontext zu stellen und automatisch auszuwerten.

Die vorliegende Erfindung zielt darauf ab, die genannten Nachteile zu überwinden und ein Verfahren bereitzustellen, das eine flexible, präzise und datenschutzkonforme Leistungsanalyse ermöglicht.

Dieses Ziel wird durch ein computerimplementiertes Verfahren zur Durchführung einer Leistungsanalyse eines ersten Benutzers gelöst, umfassend die Schritte:
- Bereitstellen einer Datenbank mit einer Vielzahl von Datensätzen, wobei jeder Datensatz eine Vielzahl von personenbezogenen Daten (die gegebenenfalls anonymisiert sind) und zumindest einen ersten Leistungsmesswert umfasst,
- Empfangen von personenbezogenen Daten des ersten Benutzers und eines gemessenen Leistungsmesswertes des ersten Benutzers oder einer Indikation eines zu messenden Leistungsmesswertes des ersten Benutzers,
- Bestimmen von zumindest zwei unterschiedlichen Untergruppen der Datenbank, wobei jede Untergruppe nur jene Datensätze der Datenbank umfasst, bei der ein ausgewähltes Datum oder eine ausgewählte Kombination von zumindest zwei Daten der personenbezogenen Daten mit einem entsprechenden Datum oder einer entsprechenden Kombination der personenbezogenen Daten des ersten Benutzers übereinstimmt oder in Verbindung steht,
- für die genannten Untergruppen, Ermitteln zumindest einer statistischen Eigenschaft, beispielsweise einer Varianz oder Standardabweichung, aus den Leistungsmesswerten der Untergruppen,
- Heranziehen jener Untergruppe, die der gemäß der zumindest einen ermittelten statistischen Eigenschaft am repräsentativsten für den ersten Benutzer ist, bevorzugt durch Durchführung einer Diskriminanzanalyse,
- Durchführen der Leistungsanalyse des ersten Benutzers durch Ermitteln eines Leistungsergebnisses des Leistungsmesswerts des ersten Benutzers innerhalb der herangezogenen Untergruppe.

Das erfindungsgemäße Verfahren ermöglicht eine Einordnung der Leistungsergebnisse des Benutzers im Kontext seiner eigenen Leistungsmöglichkeit. Konkret kann dem Benutzer sein Leistungsergebnis im Kontext jener Personengruppe angezeigt werden, die für ihn am repräsentativsten ist, und nicht etwa in einer willkürlich gewählten Gruppe. Dies ermöglicht eine gezielte Anpassung von Trainingsplänen, insbesondere, in der Physiotherapie, in der Rehabilitation und für Leistungssportler.

Als Beispiel kann vorgebracht werden, dass ein Patient einen Schlaganfall erlitten haben könnte und dadurch die Beweglichkeit einer Hand eingeschränkt ist. Mittels einer Physiotherapie kann die Beweglichkeit der Hand verbessert werden, und es ist nach dem Stand der Technik möglich, den graduellen Fortschritt zu messen (beispielsweise wird eine wöchentliche Leistungssteigerung festgestellt) - dies kann als relativer Kontext bezeichnet werden. Das erfindungsgemäße Verfahren setzt die gemessenen Leistungsmesswerte jedoch in einen absoluten Kontext bezüglich der für den Benutzer am relevantesten Personengruppe. So wird nicht etwa ein willkürlicher Gruppenbezug hergestellt, wonach die Leistung der Person im Vergleich der Personengruppe "Geschlecht: männlich" in einen absoluten Kontext gebracht wird, in welcher der Patient mit einem Schlaganfall mit einer großen Wahrscheinlichkeit im unteren Leistungsspektrum angesiedelt wäre. Erfindungsgemäß wird ein statistisches Verfahren durchgeführt, um die relevanteste Personengruppe zu bestimmen, die im vorliegenden Fall beispielsweise die Personengruppe "Krankheit: Schlaganfall" sein könnte. Das statistische Verfahren könnte beispielsweise auch als relevanteste Personengruppe jene bestimmen, die durch die Kombination der personenbezogenen Daten "Geschlecht: männlich" und "Krankheit: Schlaganfall" gebildet sein könnte. Im Kontext dieser Personengruppen kann der Patient seine Leistungsmesswerte in einem absoluten Kontext betrachten, sodass er beispielsweise ein Leistungsergebnis im oberen Drittel dieser Leistungsgruppe erreicht, während er in der Personengruppe "männlich" nur ein Leistungsergebnis im unteren Fünftel erwarten könnte. Es sei angemerkt, dass die Gruppeneinteilung umso spezifischer sein kann, je mehr Daten vorhanden sind. Beispielsweise könnte die Kombination der Daten "Krankheit: Schlaganfall", "Geschlecht: männlich", "Altersgruppe: 30-40" und "Gewichtsklasse: Adipositas 1" als relevanteste Untergruppe bestimmt werden, wenn genügend Daten vorhanden sind.

Die Einordnung der Leistungsergebnisse im absoluten Kontext können wie bereits erwähnt dabei helfen, Pläne für Trainings, Physiotherapie und Rehabilitation gezielter anzupassen. Insbesondere wird im absoluten Kontext der relevantesten Personengruppe bei Krankheiten besonders gut ersichtlich, ob die Krankheit besonders stark ausgeprägt ist, und damit beispielsweise ein sanfterer Einstieg in eine Physiotherapie oder Rehabilitation erforderlich ist, oder ob die Krankheit weniger stark ausgebildet ist und damit mit einer fortgeschrittenen Physiotherapie oder Rehabilitation begonnen werden kann.

Weiters sei angemerkt, dass die Erfindung erstmals auch ermöglicht, neuartige Zusammenhänge zwischen Leistungen und personenbezogenen Daten aufzufinden, die in der Fachwelt (z.B. dem Physiotherapeuten oder Trainer des Leistungssportlers) bislang nicht bekannt waren. So kann sich beispielsweise mit dem erfindungsgemäßen Verfahren herausstellen, dass einige Leistungsmesswerte stark durch die Kombination von bestimmten personenbezogenen Daten abhängig sind, die vorab nicht bekannt waren.

Somit können sich durch das statistische Verfahren insbesondere auch "überraschende" repräsentativste Untergruppen für den Benutzer ergeben, die ohne weiteres auch für einen geschulten Physiotherapeuten oder Trainer nicht erkennbar gewesen wären. Beispielsweise könnte ein Leistungsmesswert durch die Kombination von zwei personenbezogenen Daten besonders beeinflusst werden, wohingegen diese Kombination aus den zwei personenbezogenen Daten nur wenig Einfluss auf andere Leistungsmesswerte haben könnte. Folglich könnte das Verfahren ergeben, dass für eine weibliche Person mit einem Schlaganfall und Morbus Parkinson für einen bestimmten Leistungsmesswert die Untergruppe mit der Kombination der personenbezogenen Daten "Geschlecht: weiblich" und "Krankheit: Morbus Parkinson" die relevanteste Untergruppe ist und nicht jene mit den personenbezogenen Daten "Krankheit: Schlaganfall" (fiktives Beispiel). Es ist daher besonders vorteilhaft, wenn zumindest eine der Untergruppen, für die eine statistische Eigenschaft ermittelt wird, nur jene Datensätze der Datenbank umfasst, bei der eine ausgewählte Kombination von zumindest zwei Daten der personenbezogenen Daten mit einer entsprechenden Kombination der personenbezogenen Daten des ersten Benutzers übereinstimmt oder in Verbindung steht.

An dieser Stelle sei auch der Begriff "in Verbindung steht" erläutert. Die personenbezogenen Datensätze können beispielsweise ein genaues Geburtsdatum umfassen. Zum Zwecke der Korrelation ("in Verbindung stehen") können jedoch bestimmte Altersgruppen angenommen werden, z.B. unter 30 Jahre, 30 Jahre bis 50 Jahre und über 50 Jahre. In diesen drei Altersgruppen würden zwei Geburtsdaten, die jeweils ein Alter von über 50 Jahren angeben, miteinander in Verbindung stehen. Im Allgemeinen kann definiert werden, dass zwei personenbezogene Daten miteinander in Verbindung stehen, wenn sie in eine gemeinsame vordefinierte Klasse gruppiert werden können.

Weiters wird auch das Merkmal "Empfangen eines gemessenen Leistungsmesswertes des ersten Benutzers oder einer Indikation eines zu messenden Leistungsmesswertes des ersten Benutzers" erläutert. Für den Schritt des Bestimmens der zumindest einen Untergruppe muss eingangs noch überhaupt keine Angabe des Leistungsmesswerts bekannt sein. Für den Schritt des Ermittelns der zumindest einen statistischen Eigenschaft kann im einfachsten Fall nur eine Indikation vorliegen, in Bezug auf welche Leistungsmesswerte die statistische Eigenschaft ermittelt werden soll. Nur optional wird hierbei auch das tatsächlich gemessene Leistungsergebnis berücksichtigt. Im Schritt des Durchführens der Leistungsanalyse wird der tatsächlich gemessene Leistungsmesswert herangezogen. Um den Leistungsmesswert zu messen, kann ein Benutzer den folgenden Schritt durchführen: Durchführen einer physischen oder mentalen Übungsaufgabe und Messen des Leistungsmesswertes. Die mentale Übungsaufgabe kann beispielsweise eine Rechenaufgabe oder dergleichen sein.

Die vorliegende Erfindung ist insbesondere im Kontext von sogenannten "Serious Games" in der Medizin & Therapie anwendbar, z.B. bei Spielen zur Rehabilitation, etwa für Schlaganfallpatienten zur Verbesserung der Handmotorik. Durch das erfindungsgemäße Verfahren kann der Schwierigkeitsgrad dieser "Serious Games" gezielter eingestellt werden und dem Benutzer besseres Feedback über seinen Leistungsstand vermittelt werden.

Das ermittelte Leistungsergebnis kann an einem Bildschirm angezeigt werden und/oder einer nachfolgenden Datenverarbeitung zugeführt werden. Weiters sei angemerkt, dass das computerimplementierte Verfahren auf einem Rechner oder auf einem verteilten Rechensystem durchgeführt werden kann. Der Rechner bzw. das Rechensystem können jeweils einen oder mehrere Prozessoren zur Durchführung der benötigten Rechenschritte aufweisen und gegebenenfalls eine oder mehrere Schnittstellen aufweisen, über welche Daten eingegeben und/oder ausgegeben werden können. Der Rechner bzw. das Rechensystem kann mit der Datenbank verbunden sein oder diese umfassen. Insbesondere kann der Rechner bzw. das Rechensystem mit dem genannten Bildschirm zur Anzeige des Leistungsergebnisses verbunden sein und/oder eine Schnittstelle zum Empfang der Leistungsmesswerte und/oder der personenbezogenen Daten des ersten Benutzers (und/oder eines zweiten Benutzers) aufweisen, wobei diese Schnittstelle ein HMI (Human Machine Interface) sein kann, über welche die gemessenen Leistungsmesswerte oder die personenbezogenen Daten manuell eingegeben werden können, oder eine Schnittstelle zu einem Sensor sein kann, welcher den Leistungsmesswert unmittelbar misst.

In einer besonders bevorzugten Ausführungsform werden im Schritt des Bestimmens von zumindest zwei unterschiedlichen Untergruppen alle möglichen Untergruppen bestimmt. Dies hat den Vorteil, dass es zu keiner subjektiven "Vorselektion" kommen kann, die beispielsweise dann vorliegen würde, wenn von einer Person vorab subjektiv (und dadurch gegebenenfalls fälschlich) festgelegt wird, dass eine geschlechtsspezifische Unterscheidung keinen Einfluss auf die relevanteste Untergruppe haben wird.

In einer weiteren bevorzugten Variante wird eine statistische Eigenschaft nur dann für eine zuvor bestimmte Untergruppe ermittelt, wenn die Untergruppe statistisch relevant ist, insbesondere wenn die Untergruppe eine vorbestimmte Anzahl von Datensätzen umfasst. In anderen Worten wird zuerst eine Untergruppe erstellt und dann geprüft, ob diese statistisch relevant ist. Wenn eine Untergruppe nur wenige Einträge umfasst, kann prinzipiell davon ausgegangen werden, dass diese nicht statistisch relevant sein wird, und daher wird diese Untergruppe von der weiteren Verarbeitung ausgeschlossen. Dieser Schritt wird insbesondere dann relevant sein, wenn zuvor alle möglichen Untergruppen bestimmt wurden, da manche Kombinationen von persönlichen Daten nur selten auftreten und daher statistisch nicht relevant sein werden. Es versteht sich, dass die statistische Relevanz auch anders bestimmt werden kann. Im Allgemeinen kann angemerkt werden, dass die statistische Relevanz die Wahrscheinlichkeit beschreibt, dass ein beobachteter Effekt nicht nur zufällig aufgetreten ist, sondern tatsächlich eine systematische Ursache hat. Sie kann daher typischerweise durch Signifikanztests bestimmt werden, die prüfen, ob ein Effekt (z. B. ein Unterschied zwischen zwei Gruppen oder ein Zusammenhang zwischen Variablen) mit hoher Wahrscheinlichkeit in der Grundgesamtheit existiert und nicht nur auf Zufallsschwankungen in der Stichprobe zurückzuführen ist. Im vorliegenden Kontext kann die statistische Relevanz beispielsweise über die Stichprobengröße und/oder über die Effektgröße bestimmt werden. Die Stichprobengröße beeinflusst die Aussagekraft der Analyse, da größere Stichproben zufällige Schwankungen reduzieren und kleinere Effekte erkennbar machen. Die Effektgröße gibt die Stärke eines beobachteten Effekts an, wobei kleine Effekte trotz Signifikanz praktisch irrelevant sein können, während große Effekte auch mit kleineren Stichproben nachweisbar sind.

Besondere Maßnahmen können dann getroffen werden, wenn zwei verschiedene Leistungsmesswerte desselben Benutzers vorliegen. Beispielsweise hat ein Benutzer die persönlichen Daten "Krankheit: Schlaganfall" und "Alter: über 50" und beginnt eine Leistungsfeststellung, wie z.B. das Spielen eines Kugellabyrinths. Hierbei können die Leistungsmesswerte beispielsweise "benötigte Zeit" und "zurückgelegte Wegstrecke der Kugel" sein. Für den ersten Leistungsmesswert "benötigte Zeit" werden die Untergruppen "Krankheit: Schlaganfall" und "Alter: über 50" analysiert und es wird festgestellt, dass die relevanteste Untergruppe jene mit dem Datum "Krankheit: Schlaganfall" ist. Weiters werden für den zweiten Leistungsmesswert "zurückgelegte Wegstrecke der Kugel" beide genannten Untergruppen analysiert und es wird festgestellt, dass die relevanteste Untergruppe jene mit "Alter: über 50" ist, da beispielsweise die Krankheit Schlaganfall keine Auswirkung auf die zurückgelegte Wegstrecke hat und das Alter relevanter für die zurückgelegte Wegstrecke ist (fiktives Beispiel). Es ist ersichtlich, dass das erfindungsgemäße Verfahren somit auch eine individuelle Analyse von unterschiedlichen Leistungsmesswerten ermöglicht. Bevorzugt umfasst das Verfahren somit die folgenden Schritte:
- Empfangen eines gemessenen zweiten Leistungsmesswertes des ersten Benutzers oder Empfangen einer Indikation eines zu messenden zweiten Leistungsmesswertes des ersten Benutzers,
- Wiederholen der Schritte Ermitteln, Heranziehen und Durchführen für den zweiten Leistungsmesswert.

In der genannten Variante ist bevorzugt, wenn für die beiden Leistungsmesswerte unterschiedliche Untergruppen herangezogen werden, wobei sich die Untergruppen besonders bevorzugt überlappen. Im genannten Beispiel mit den Untergruppen "Krankheit: Schlaganfall" und "Alter: über 50" kann dies beispielsweise der Fall sein, da in der Untergruppe "Krankheit: Schlaganfall" auch Personen angeführt sein können, bei denen das Kriterium "Alter: über 50" angeführt ist.

In einer weiteren bevorzugten Ausführungsform kann eine weitere Untergruppe herangezogen werden und eine weitere Leistungsanalyse des ersten Benutzers durch Ermitteln eines weiteren Leistungsergebnisses des Leistungsmesswerts innerhalb der weiteren herangezogenen Untergruppe durchgeführt werden. Diese weitere Untergruppe muss nicht die relevanteste Untergruppe sein, sondern beispielsweise eine vom Benutzer ausgewählte Untergruppe. Insbesondere können in dieser Variante beide Leistungsergebnisse als vergleichende Darstellung auf einem Bildschirm angezeigt werden. Dadurch ist dem Benutzer ersichtlich, wie sein Leistungsergebnis in Bezug auf alle Personen mit Schlaganfall aussieht (relevanteste Gruppe), und wie sein Leistungsergebnis in Bezug auf alle männlichen Personen aussieht (vom Benutzer gewählte weitere Untergruppe). Die weitere Untergruppe kann beispielsweise auch ein Trainingsziel sein, sodass der Benutzer weiß, wie er seine Leistung steigern muss, um ein Normalziel zu erreichen. Bevorzugt enthält die relevanteste Untergruppe die Angabe, dass eine spezifische Krankheit vorliegt, und die weitere Untergruppe enthält die Angabe, dass die spezifische Krankheit nicht vorliegt.

Besonders bevorzugt kann das erfindungsgemäße Verfahren auch dazu eingesetzt werden, um Leistungsergebnisse von zwei Benutzern zu vergleichen, die an sich unterschiedliche Leistungsmaxima haben, z.B. weil einer der Benutzer eine bestimmte Krankheit hat oder besonders jung oder alt ist. Um dies umzusetzen, kann das Verfahren die Schritte Empfangen, Bestimmen, Ermitteln, Heranziehen und Durchführen für einen zweiten Benutzer wiederholen, wobei der Typ des Leistungsmesswertes des ersten Benutzers dem Typ des Leistungsmesswertes des zweiten Benutzers entspricht (d.h. die beiden Benutzer haben dieselbe Übung durchgeführt und es wurde derselbe Leistungsmesswert gemessen, z.B. eine "benötigte Zeit"), und wobei die herangezogenen Untergruppen für den ersten Benutzer und den zweiten Benutzer unterschiedlich sind. In einem Beispiel könnte ein erster Benutzer einen Schlaganfall erlitten haben, sodass die für ihn relevante Untergruppe jene mit dem persönlichen Datum "Krankheit: Schlaganfall" ist. Der zweite Benutzer könnte ein Jugendlicher sein, sodass die für ihn relevante Untergruppe jene mit dem persönlichen Datum "Alter: unter 30 Jahre" ist. Beide Benutzer spielen nun z.B. ein Kugellabyrinth, bei dem der Leistungsmesswert die "benötigte Zeit" ist. Den Benutzern wird nun ihr eigenes Leistungsergebnis in Relation zu der für sie relevantesten Untergruppe angezeigt und können somit ihre beiden Messwerte vergleichen. Beispielsweise hat der erste Benutzer eine längere Zeit benötigt, um das Kugellabyrinth zu absolvieren, jedoch ist das Leistungsergebnis des ersten Benutzers in seiner relevantesten Untergruppe 20% über dem Durchschnitt und das Leistungsergebnis des zweiten Benutzers in seiner relevantesten Untergruppe 10% über dem Durchschnitt. In diesem Fall würde der erste Benutzer trotz der längeren benötigten Zeit den Vergleich gewinnen. Dieses Verfahren ist besonders für Patienten in einer Therapie oder Rehabilitation bevorzugt, da der direkte Vergleich mit anderen Personen besonders motivierend ist, und kann dadurch die Therapie oder Rehabilitation beschleunigen. Da sich ein Patient durch Genesung zudem verbessert, kann davon ausgegangen werden, dass sich seine Leistungsergebnisse stetig verbessern. Es kann dadurch vorkommen, dass zu einem späteren Zeitpunkt eine andere Untergruppe als relevanteste Untergruppe herangezogen werden kann. Dadurch kann eine relative Schwierigkeit konstant gehalten werden, was eine zusätzliche Motivation darstellen kann.

Im genannten Verfahren ist besonders bevorzugt, wenn die Leistungsmesswerte des ersten Benutzers und des zweiten Benutzers im Wesentlichen zeitgleich ermittelt werden und die Leistungsergebnisse des ersten Benutzers und des zweiten Benutzers bevorzugt als vergleichende Darstellung auf einem Bildschirm angezeigt werden. Dadurch kann ein direkter Wettkampf der beiden Benutzer vor Ort stattfinden, was besonders für "Serious Games" förderlich und motivierend ist.

Es versteht sich, dass die eingangs erwähnte Datenbank möglichst viele Datensätze umfasst. Um die Datenbank ständig zu erweitern, wird vorgesehen, dass die personenbezogenen Daten und die gemessenen Leistungsmesswerte der Datenbank hinzugefügt werden, nachdem die Leistungsanalyse durchgeführt wurde. Es können jedoch auch Kriterien vorgesehen werden, um nur ausgewählte Datensätze in die Datenbank aufzunehmen. Dadurch können beispielsweise Messwerte, die offensichtliche Messfehler oder auf willkürlich verfälschten Leistungen basieren, ausgefiltert werden, um die Datenbank nicht zu verfälschen.

In einer ersten Variante ist bevorzugt, wenn zumindest eine der ermittelten statistischen Eigenschaften eine Varianz oder Standardabweichung der Leistungsmesswerte der Untergruppen ist und eine Untergruppe umso repräsentativer ist, desto geringer ihre Varianz oder Standardabweichung ist. Wenn die Varianz oder Standardabweichung die einzige ermittelte statistische Eigenschaft ist, wird die Untergruppe mit der geringsten Varianz oder Standardabweichung als repräsentativste Untergruppe ausgewählt.

In einer zweiten Variante kann zumindest eine der ermittelten statistischen Eigenschaften vom Leistungsmesswert des ersten Benutzers abhängig sein, wobei die genannte statistische Eigenschaft bevorzugt der Abstand des Leistungsmesswert des ersten Benutzers zum Mittelwert der Leistungsmesswerte der Untergruppe und eine Untergruppe umso repräsentativer ist, desto geringer der Abstand des Leistungsmesswert des ersten Benutzers zum Mittelwert der Leistungsmesswerte der Untergruppe ist. Wenn der Abstand die einzige statistische Eigenschaft ist, wird die Untergruppe mit dem geringsten Abstand als repräsentativste Untergruppe ausgewählt.

Bevorzugt werden jedoch zumindest zwei statistische Eigenschaften ermittelt und die repräsentativste Untergruppe als Funktion aller ermittelten statistischen Eigenschaften bestimmt. An dieser Stelle sei festgehalten, dass dieses Verfahren jedoch auch mittels eines Maschinenlernalgorithmus ausgeführt werden kann und eine analytische Bestimmung der repräsentativsten Untergruppe nicht immer notwendig ist.

Das vorgenannte System kann beispielsweise anhand zumindest einer Recheneinheit oder allgemeiner einer Vorrichtung zur Datenverarbeitung ausgebildet sein, die dazu ausgebildet ist, das vorgenannte Verfahren durchzuführen.

Im Folgenden werden bevorzugte und nicht einschränkende Ausführungsformen durch Verweis auf die angehängten Zeichnungen erläutert.
Figur 1 zeigt eine Datenbank mit einer Vielzahl von Datensätzen und einen Benutzer.
Figur 2 zeigt einen ersten Unterdatensatz der Datenbank.
Figur 3 zeigt einen zweiten Unterdatensatz der Datenbank.
Figur 4 zeigt einen dritten Unterdatensatz der Datenbank.
Figur 5 zeigt eine Verteilung der Leistungsmesswerte innerhalb des ersten Unterdatensatzes.
Figur 6 zeigt eine Verteilung der Leistungsmesswerte innerhalb des zweiten Unterdatensatzes.
Figur 7 zeigt eine Verteilung der Leistungsmesswerte innerhalb des dritten Unterdatensatzes.
Figur 8 zeigt für die Untergruppen bestimmte Repräsentationsfaktoren.

Gemäß dem vorliegenden Verfahren wird eingangs eine Datenbank 1 bereitgestellt, in der eine Vielzahl von Datensätzen DS1, DS2, ... DSi gespeichert ist. Die Datenbank 1 kann Festplatten, SSDs oder einen Arbeitsspeicher umfassen. Die Datenbank 1 kann insbesondere als dedizierter Server oder auch in der Cloud verwirklicht sein.

Im dargestellten Beispiel umfasst jeder Datensatz DSi zwei personenbezogene Daten pD und zwei Leistungsmesswerte LM. Personenbezogene Daten pD sind alle Informationen, die sich auf eine natürliche Person beziehen. Im Allgemeinen kann jeder Datensatz DSi zumindest ein oder zumindest zwei personenbezogene Daten pD und zumindest einen oder zumindest zwei Leistungsmesswerte LM umfassen.

Die personenbezogenen Daten pD können anonymisiert sein und dadurch nicht einer Person zugeordnet werden. Personenbezogene Daten pD können beispielsweise ein Geburtsdatum oder Alter, Gesundheitsdaten (z. B. Gewicht, Diagnosen, medizinische Befunde), genetische Daten oder dergleichen umfassen. Die Gesundheitsdaten können insbesondere eine Angabe über eine Krankheit (z.B. die Angabe "Krankheit X liegt vor" oder "Krankheit Y liegt nicht vor") sein. Im Allgemeinen ist bevorzugt, wenn jeder Datensatz DSi so viele personenbezogene Daten pD wie möglich umfasst.

Die Leistungsmesswerte LM sind Messdaten, welche die Ergebnisse von bestimmten Übungen sind. Beispielsweise kann ein Leistungsmesswert die Anzahl der erfolgreich gemachten Liegestütze und/oder die Anzahl der erfolgreich gemachten Kniebeugen sein. Die Leistungsmesswerte LM können auch mittels Hilfsmittel gemessen werden, z.B. mittels eines Kugellabyrinths, bei dem eine "benötigte Zeit" und "zurückgelegte Wegstrecke der Kugel" gemessen werden. Aus diesen zwei Beispielen ist ersichtlich, dass die Leistungsmesswerte LM eines Datensatzes DSi einer Person aus verschiedenen Übungen gewonnen wurden oder dass die Leistungsmesswerte LM eines Datensatzes DSi einer Person aus derselben Übung gewonnen wurden. Eine entsprechende Mischung von Leistungsmesswerten ist möglich und im Allgemeinen ist bevorzugt, wenn jeder Datensatz DSi so viele Leistungsmesswerte LM wie möglich umfasst.

Für das hierin beschriebene Verfahren sollen anhand der in der Datenbank 1 hinterlegten Datensätze DSi die Leistungsmesswerte LMB1 eines ersten Benutzers B1 evaluiert werden, sodass diese als Leistungsergebnis in Bezug auf andere Personen ausgegeben werden. In einem anfänglichen Schritt reicht es, wenn die personenbezogenen Daten pdB 1 des ersten Benutzers B1 erhalten werden und erst danach die tatsächlich gemessenen Leistungsmesswerte LMB 1 oder eine Indiktion darüber, welche Leistungsmesswerte LMB1 gemessen werden sollen. Im vorliegenden Beispiel werden zwei personenbezogene Daten pDB1-1 und pdB 1-2 des ersten Benutzers B1 erhalten, wobei hier pDB 1-1 die Indikation ist, dass eine Krankheit vorliegt, und pdB1-2 das Gewicht des ersten Benutzers B1 ist.

Anhand der personenbezogenen Daten pDB 1-1 und pDB1-2 des ersten Benutzers B1 sollen nun aus der Datenbank DB jene Untergruppen von Datensätzen DSi extrahiert werden, die besonders repräsentativ für den ersten Benutzer B1 sind. Hierfür werden die Datensätze DSi in der Datenbank DB zuerst in mehrere Untergruppen aufgeteilt.

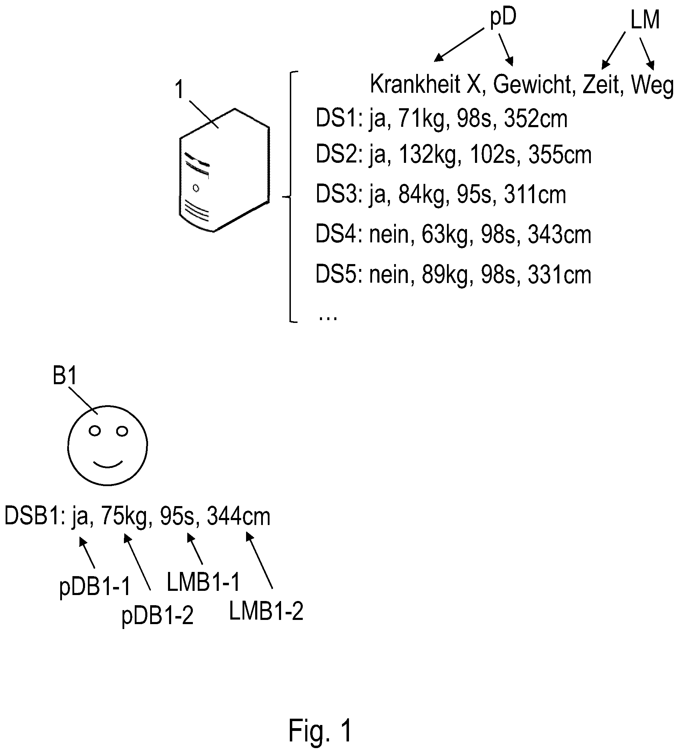

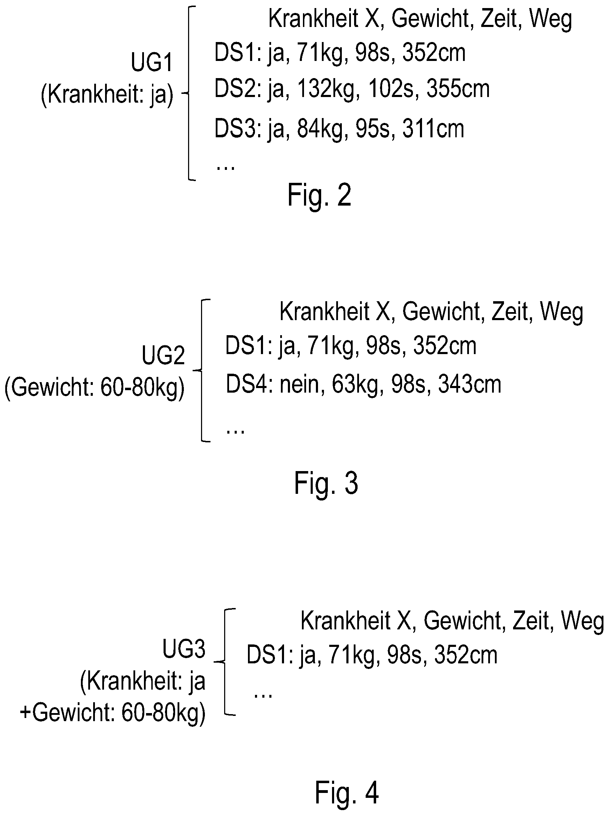

Die Figuren 2, 3 und 4 zeigen Untergruppen, die aus den Datensätzen DSi der Datenbank 1 für den ersten Benutzer B1 erstellt werden. Für dieses Beispiel sei angemerkt, dass der erste Benutzer B1 in Figur 1 die personenbezogenen Daten "Krankheit X: ja" und "Gewicht: 75 kg" angegeben hat.

Die Untergruppen werden bestimmt, indem ein Datum der personenbezogenen Daten des ersten Benutzers B1 herangezogen wird, z.B. "Krankheit X: ja". Danach werden alle Datensätze DSi aus der Datenbank gesucht, die ebenfalls das genannte Datum aufweisen, d.h. im genannten Beispiel "Krankheit X: ja". Für die in Figur 1 dargestellte Datenbank 1 wären das zumindest die Datensätze DS1, DS2 und DS3, aber nicht die Datensätze DS4 und DSi. Diese Datensätze DS1, DS2 und DS3 bilden eine erste Untergruppe UG1, da sie nur jene Datensätze DSi der Datenbank 1 umfasst, bei denen das ausgewählte Datum "Krankheit X: ja" mit dem entsprechenden Datum "Krankheit X: ja" der personenbezogenen Daten pDB 1 des ersten Benutzers B1 übereinstimmt.

Eine zweite Untergruppe kann gebildet werden, indem ein anderes Datum der personenbezogenen Daten des ersten Benutzers B1 herangezogen wird, z.B. "Gewicht: 75 kg". Es ist ersichtlich, dass kein anderes Gewicht in der Datenbank 1 genau "75 kg" ist und es für die Auswertung vielmehr relevant ist, ob das Gewicht ähnlich ist. Aus diesem Grund sollen diese personenbezogenen Daten lediglich in Verbindung stehen. Hierfür werden eingangs hilfsweise diskrete Wertebereiche angenommen und es wird geprüft, welche Datensätze DSi in der Datenbank 1 ein personenbezogenes Datum aufweisen, das im selben diskreten Wertebereich liegt wie das entsprechende Datum der personenbezogenen Daten pDB 1 des ersten Benutzers B1, d.h. mit diesem Datum der personenbezogenen Daten pDB 1 des ersten Benutzers B1 in Verbindung steht. Dies ist in Figur 3 durch den diskreten Wertebereich "60-80 kg" dargestellt, sodass das entsprechende personenbezogene Datum der Datensätze DS1 und DS4 der Datenbank 1 von Figur 1 im selben Wertebereich wie das personenbezogene Datum des ersten Benutzers B1 vorliegt.

Weiters können entsprechende Untergruppen gefunden werden, bei der die Untergruppe nur jene Datensätze DSi der Datenbank 1 umfasst, bei der eine ausgewählte Kombination von zumindest zwei Daten der personenbezogenen Daten mit einer entsprechenden Kombination der personenbezogenen Daten des ersten Benutzers übereinstimmt oder korreliert. Im Fall von Figur 1 wird eine Kombination der personenbezogenen Daten "Krankheit X: ja" und "Gewicht: 75 kg" des ersten Benutzers B1 herangezogen. Es wird in der Datenbank 1 gesucht, bei welchen Datensätzen DSi sowohl "Krankheit X: ja" vorhanden ist, als auch "Gewicht: 60-80 kg" (wie oben erläutert können auch hier Daten mithilfe eines diskreten Wertebereichs korreliert werden). Im Beispiel der Figur 1 ist dies lediglich der Datensatz DS1, wie in Figur 4 detailliert dargestellt ist.

Mit diesem Verfahren wurden aus der Datenbank 1 drei Untergruppen UG1, UG2, UG3 bestimmt, die jeweils für den Benutzer 1 repräsentativ sind. Es versteht sich, dass auch viel mehr Untergruppen erstellt werden können, z.B. wenn mehr als zwei personenbezogene Daten pDB 1 des ersten Benutzers B1 vorhanden sind und dadurch mehr Untergruppen mit einzeln übereinstimmenden oder korrelierenden personenbezogenen Daten oder mehr Untergruppen mit Kombinationen von übereinstimmenden oder korrelierenden personenbezogenen Daten bestimmt werden können. Es versteht sich natürlich, dass auch Untergruppen mit Kombinationen aus mehr als zwei übereinstimmenden oder korrelierenden personenbezogenen Daten gebildet werden können.

In der Regel werden alle möglichen Untergruppen bestimmt, wobei dies nicht notwendig ist, z.B. wenn augenscheinlich ist, dass eine Untergruppe statistisch keinen Sinn mach, z.B. weil sie zu eng gestreut ist und dadurch zu wenige Datensätze DSi in der Untergruppe vorhanden sein werden oder zu breit ist und dadurch keine statistisch relevante Aussage getroffen werden kann. Bei den Untergruppen kann somit vorab noch geprüft werden, ob diese statistisch relevant ist, z.B. ob diese Untergruppe eine vorbestimmte Anzahl von Datensätzen umfasst oder zu generisch ist.

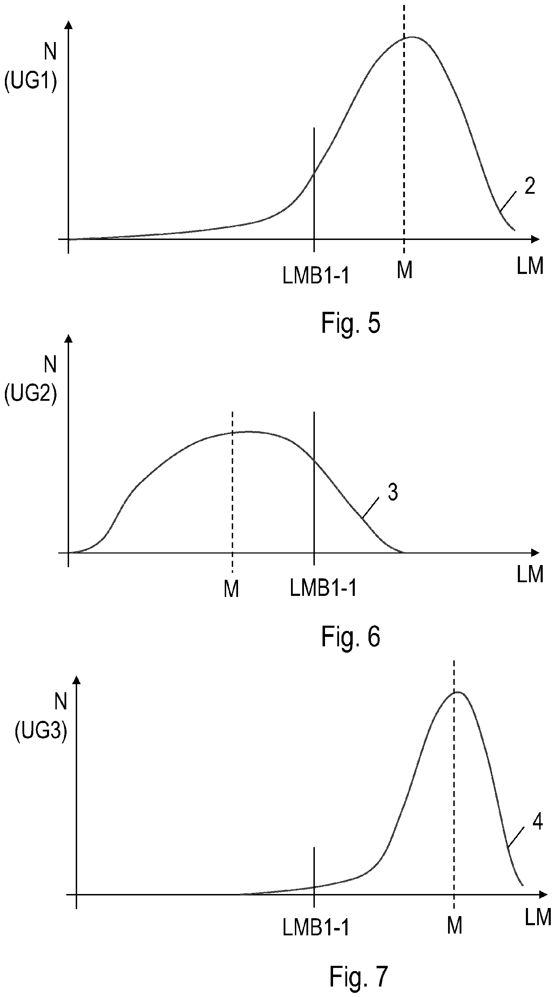

Im nächsten Verfahrensschritt wird für die Untergruppen eine statistisch relevante Eigenschaft ermittelt, um daraus ein Leistungsergebnis für den ersten Leistungsmesswert LMB 1-1 zu ermitteln (der zweite Leistungsmesswert LMB 1-2 des ersten Benutzers wird vorerst aus Gründen der leichteren Erklärung unberücksichtigt gelassen). Dies wird anhand der Figuren 5, 6 und 7 erläutert.

Die Figuren 5, 6 und 7 zeigen jeweils eine Verteilung der in der Datenbank 1 angeführten ersten Leistungsmesswerte LM (die vom selben Typ sind wie der zu untersuchende erste Leistungsmesswert LMB1-1 des ersten Benutzers B1) der Datensätze DSi der Untergruppen UG1, UG2, UG3, wobei auf der vertikalen Achse die Anzahl N der Datensätze DSi angeführt ist und auf der horizontalen Achse der jeweilige Leistungsmesswert. Hierbei wurde angenommen, dass in der Datenbank 1 weitaus mehr Datensätze DSi hinterlegt sind als in Figur 1 dargestellt, und über die diskreten Werte wurden die dargestellten Verteilungskurven 2, 3 und 4 gezogen. Somit gibt die Verteilungskurve 2 die Verteilung der ersten Leistungsmesswerte der ersten Untergruppe UG1 an, die zweite Verteilungskurve 3 die Verteilung der ersten Leistungsmesswerte der zweiten Untergruppe UG2 an und die Verteilungskurve 3 die Verteilung der ersten Leistungsmesswerte der dritten Untergruppe UG3 an.

In einem ersten Beispiel wird die statistische Eigenschaft der Untergruppe als Varianz oder Standardabweichung (Streuung) der jeweiligen Untergruppe bestimmt. Die Varianz ist ein statistisches Maß, das angibt, wie stark die Werte eines Datensatzes um ihren Mittelwert M streuen. Sie misst also, wie weit die einzelnen Datenpunkte im Durchschnitt vom Mittelwert M entfernt liegen. Die Standardabweichung ist die Wurzel der Varianz. Aus dem Vergleich der Verteilungskurven 2, 3 und 4 ist ersichtlich, dass die Verteilungskurve 3 die größte Streuung aufweist. Es kann daher darauf geschlossen werden, dass die Untergruppe UG2 in diesem Beispiel nicht als repräsentativste Untergruppe angesehen wird. Es verbleiben die Untergruppen UG1, UG3, wobei die Untergruppe UG3 eine geringfügig kleinere Streuung als die erste Untergruppe UG1 aufweist und daher in diesem Beispiel als repräsentativste Untergruppe angesehen wird.

Als Hintergrund für die Verwendung der Streuung als statistische Eigenschaft kann erwähnt werden, dass z.B. bei einer Krankheit ähnliche Symptome vorliegen können, die das Leistungsvermögen alle von der Krankheit betroffenen Benutzer in einer gleichen Art und Weise einschränken wird, z.B. bei einer partiellen Lähmung einer Hand.

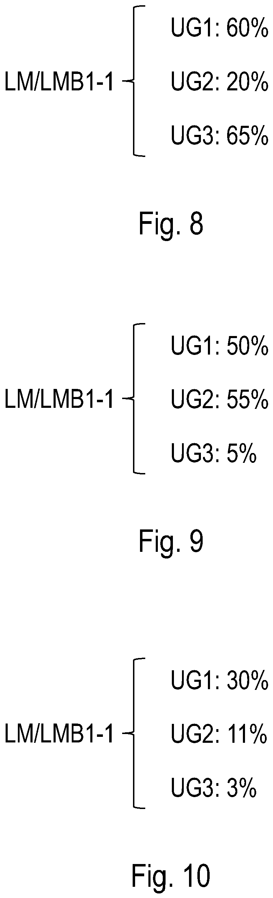

Figur 8 zeigt für dieses erste Beispiel, dass in Bezug auf einen Leistungsmesswert LM bzw. LMB 1-1 für jede der Untergruppen UG1, UG2, UG3 ein Repräsentationsfaktor ermittelt werden kann. Im ersten Beispiel ist der Repräsentationsfaktor z.B. der Kehrwert der Streuung. Die repräsentativste Untergruppe ist jene mit dem größten Repräsentationsfaktor, d.h. im ersten Beispiel die dritte Untergruppe UG3.

Aus dem genannten ersten Beispiel ist ersichtlich, dass die statistische Eigenschaft ohne Berücksichtigung des ersten Leistungsmesswerts LMB 1-1 des ersten Benutzers B1 ermittelt wird. Es kann jedoch auch eine statistische Eigenschaft unter Berücksichtigung des ersten Leistungsmesswerts LMB 1-1 des ersten Benutzers B1 ermittelt werden. Insbesondere kann die statistische Eigenschaft in einem zweiten Beispiel ein Abstand des ersten Leistungsmesswertes LMB 1-1 zum Mittelwert M in der jeweiligen Untergruppe sein. Demzufolge gibt ein großer Abstand des ersten Leistungsmesswertes LMB1-1 zum Mittelwert M an, dass es unwahrscheinlich ist, dass der Benutzer 1 dieser Untergruppe zugehörig ist, wie insbesondere aus Figur 7 ersichtlich ist. Wenn nur diese statistische Eigenschaft herangezogen wird, wäre die zweite Untergruppe UG2 die für den ersten Benutzer B1 repräsentativste Untergruppe.

Als Hintergrund für die Verwendung des Abstands des ersten Leistungsmesswertes LMB1-1 zum Mittelwert M kann erwähnt werden, dass z.B. Symptome von Krankheiten durch eine Therapie oder Rehabilitation auch überwunden können werden, sodass ein geheilter Benutzer mit einem Schlaganfall in der Vergangenheit auch eine Leistung erzielen kann, die nicht mehr mit Leistungen klassischer Schlaganfallpatienten vergleichbar ist. Ein geheilter Benutzer mit einem Schlaganfall in der Vergangenheit kann in seinem Leistungsvermögen somit auch wieder einer breiten Personengruppe zugeordnet werden.

Figur 9 zeigt für dieses zweite Beispiel, dass in Bezug auf einen Leistungsmesswert LM bzw. LMB 1-1 für jede der Untergruppen UG1, UG2, UG3 ein Repräsentationsfaktor ermittelt werden kann. Im zweiten Beispiel ist der Repräsentationsfaktor z.B. der Kehrwert des Abstands des ersten Leistungsmesswertes LMB 1-1 zum Mittelwert M. Die repräsentativste Untergruppe ist jene mit dem größten Repräsentationsfaktor, d.h. im zweiten Beispiel die zweite Untergruppe UG2.

Um beide Aspekte zu berücksichtigen, werden zumindest zwei statistische Eigenschaften der Untergruppen ermittelt, wobei optional eine erste statistische Eigenschaft auf Basis des ersten Leistungsmesswerts LMB 1-1 des ersten Benutzers B1 ermittelt wird und die zweite statistische Eigenschaft ohne Berücksichtigung des ersten Leistungsmesswerts LMB 1-1 des ersten Benutzers B1 ermittelt wird. Beispielsweise kann sowohl die Streuung als auch der Abstand des ersten Leistungsmesswertes LMB1-1 zum Mittelwert M ermittelt werden. Die repräsentativste Untergruppe kann auf Basis aller ermittelten statistischen Eigenschaften bestimmt werden, sodass eine Abwägung der obigen Erkenntnisse getroffen wird und die erste Untergruppe UG1 als repräsentativste Untergruppe bestimmt werden kann, da einerseits die Streuung nicht zu groß ist und andererseits auch der Abstand zum Mittelwert auf ein plausibles Ergebnis hindeutet.

Figur 10 zeigt für dieses dritte Beispiel, dass in Bezug auf einen Leistungsmesswert LM bzw. LMB 1-1 für jede der Untergruppen UG1, UG2, UG3 ein Repräsentationsfaktor ermittelt werden kann. Im dritten Beispiel ist der Repräsentationsfaktor z.B. der Kehrwert der Streuung (Figur 8) multipliziert mit dem Kehrwert des Abstands des ersten Leistungsmesswertes LMB 1-1 zum Mittelwert M (Figur 9). Es versteht sich jedoch, dass der Repräsentationsfaktor durch andere mathematische Modelle errechnet werden kann. Die repräsentativste Untergruppe ist jene mit dem größten Repräsentationsfaktor, d.h. im dritten Beispiel die erste Untergruppe UG1.

An dieser Stelle sei festgehalten, dass anhand der ermittelten Repräsentationsfaktoren auch Rückschlüsse auf wichtige Leistungsfaktoren geschlossen werden können. Beispielweise hätte bei der Berechnungsmethode aus Figur 8 die Krankheit einen Einfluss von 60% auf den Leistungsmesswert und das Gewicht einen Einfluss von 20% auf den Leistungsmesswert. Es kann daher bevorzugt sein, die Repräsentationsfaktoren für zumindest eine, zumindest zwei oder alle Untergruppen auszugeben bzw. anzuzeigen, insbesondere auf einem Bildschirm anzuzeigen.

Weiters sei erwähnt, dass das Verfahren nicht so analytisch durchgeführt werden muss, wie es in den obigen Absätzen beschrieben wurde. Es kann insbesondere auch eine Diskriminanzanalyse durchgeführt werden, optional unter Verwendung von Maschinenlernalgorithmen, um zu bestimmen, welche Untergruppe am repräsentativsten für den jeweiligen Benutzer ist.

Mit den obigen Erwägungen kann somit aus den statistischen Eigenschaften die repräsentativste Untergruppe bestimmt werden. Im weiteren Verfahren wird ein Leistungsergebnis des ersten Benutzers innerhalb der herangezogenen Untergruppe ermittelt. Das Leistungsergebnis kann beispielsweise die Aussage "X% besser oder schlechter als der Mittelwert in dieser Untergruppe" oder "Y Sekunden schneller oder langsamer als der Mittelwert in dieser Untergruppe" sein. Dies gibt dem Benutzer eine Auskunft über seine Leistung im Vergleich zu anderen Benutzern im selben Leistungsspektrum, z.B. kann bei einem Patienten, die kürzlich einen Schlaganfall erlitten haben, die Schwere der Symptome objektiv ermittelt werden. Weiters kann dieses Leistungsergebnis dazu eingesetzt werden, um Physiotherapien und Rehabilitationen entsprechend an den Benutzer B1 anzupassen.

Bislang wurde nur das Verfahren zur Bestimmung eines Leistungsergebnisses für den ersten Leistungsmesswert LMB1-1 erläutert. Wie in Figur 1 dargestellt kann jedoch auch ein zweiter Leistungsmesswert LMB1-2 für den ersten Benutzer B1 vorliegen. Das vorgenannte Verfahren kann wiederholt werden, sodass unabhängig von den vorhergegangenen Schritten des Ermittelns der statistischen Eigenschaft aus den ersten Leistungsmesswerten der Untergruppen statistische Eigenschaften aus den zweiten Leistungsmesswerten der Untergruppen ermittelt werden können. Dies kann anhand derselben Überlegungen wie oben durchgeführt werden, jedoch kann das Verfahren zu dem Ergebnis kommen, dass in Bezug auf den zweiten Leistungsmesswert LMB 1-2 eine andere Untergruppe für den Benutzer B1 am repräsentativsten ist als in Bezug auf den ersten Leistungsmesswert LMB 1-1. Dies kann damit zu tun haben, dass z.B. eine erste Leistungsübung für einen Schlaganfallpatienten schwer durchführbar ist, aber eine zweite Leistungsübung im selben Maße durchgeführt werden kann.

Alternativ kann jedoch vorgesehen werden, dass zumindest eine statistische Eigenschaft für die Untergruppe in Bezug auf zumindest zwei Leistungsmesswerte ermittelt wird. In diesem Fall wird keine "einfache" zweidimensionale Darstellung von Verteilungskurven wie in den Figuren 5 bis 7 darstellbar sein, sondern es wird die statistische Eigenschaft auf Basis von Verteilungen in einem multidimensionalen Raum ermittelt. In anderen Worten wird die statistische Eigenschaft als Funktion von ersten Leistungsmesswerten und zweiten Leistungsmesswerten aus den Untergruppen erstellt. Anschließend kann wiederum die für den Benutzer B1 repräsentativste Untergruppe bestimmt werden, analog zu den obigen Ausführungen, wobei die Ermittlung der repräsentativsten Untergruppe in einem multidimensionalen Raum stattfindet. Insbesondere in diesem Fall bietet sich eine Diskriminanzanalyse mittels Maschinenlernalgorithmen an.

Aus den Figuren 2 bis 4 ist insbesondere ersichtlich, dass sich die Untergruppen auch überlappen können, was bedeutet, dass gleiche Datensätze DSi in zumindest zwei Untergruppen vorkommen.

Mit dem obigen Verfahren kann wie erläutert die für den Benutzer repräsentativste Untergruppe gefunden werden, damit die Leistung des Benutzers z.B. mit anderen Schlaganfallpatienten verglichen werden kann. Oft kann jedoch gewünscht sein, dass dem Benutzer auch seine "Zielgruppe" angezeigt wird, die zwar im derzeitigen Moment für den Benutzer B1 noch nicht repräsentativ ist, jedoch als Zielvorlage dienen soll, die der Benutzer erreichen soll. Als Beispiel wird auf die Figuren 5 und 6 verwiesen. Gemäß dem obigen Verfahren wurde die erste Untergruppe als repräsentativste Untergruppe ermittelt, in welcher der Benutzer ein Leistungsergebnis von "besser als 90% der Schlaganfallpatienten" aufweist. Das Ziel ist jedoch, dass der Benutzer in seiner Gewichtsklasse "60-80kg" der zweiten Untergruppe überdurchschnittlich abschneidet, in welcher der erste Benutzer B1 derzeit jedoch nur ein Leistungsergebnis von "besser als 40% der Benutzer mit 60-80kg" erreicht. Dies dient insbesondere in der Physiotherapie und Rehabilitation als Motivation zur Genesung. Die beiden Leistungsergebnisse können insbesondere als vergleichende Darstellung auf einem Bildschirm angezeigt werden.

Das genannte Verfahren kann weiters für einen zweiten Benutzer durchgeführt werden, wobei der zweite Benutzer eingangs auch seine eigenen persönlichen Daten zur Verfügung stellt. Der erste Benutzer B1 und der zweite Benutzer führen gegebenenfalls gleichzeitig (oder zeitverzögert) dieselbe Übung durch und erhalten dadurch einen oder mehrere eigene Leistungsmesswerte. Hierbei entspricht der Typ des Leistungsmesswertes des ersten Benutzers B1 dem Typ des Leistungsmesswertes des zweiten Benutzers, d.h. es wird dieselbe Eigenschaft gemessen, z.B. eine Zeit einer bestimmten Übung oder dergleichen. Das oben erläuterte Verfahren wird für beide Benutzer unabhängig durchgeführt, sodass für jeden der Benutzer eine eigene Untergruppe gefunden wird, die jeweils am repräsentativsten für den jeweiligen Benutzer ist. Dadurch können die Leistungsergebnisse des ersten Benutzers und des zweiten Benutzers beispielsweise als vergleichende Darstellung auf einem Bildschirm angezeigt werden und es wird angezeigt, wie gut der jeweilige Benutzer im Vergleich zu der jeweils für ihn repräsentativsten Gruppe von Benutzern abgeschnitten hat.

Nach oder schon vor Abschluss des Verfahrens kann der in Figur 1 dargestellte Datensatz DSB 1 des ersten Benutzers B1 der Datenbank 1 hinzugefügt werden, um diese entsprechend zu erweitern.

Die obigen Beispiele wurden insbesondere für den Gesundheitsbereich erläutert, d.h. zumindest eine der personenbezogenen Daten betrifft eine Angabe zu einer Krankheit oder zu einem Symptom des Benutzers. Die Datenbank 1 umfasst dabei zumindest zwei, bevorzugt über hundert, Datensätze von Personen, die welche dieselbe Krankheit oder dasselbe Symptom wie der erste Benutzer B1 aufweisen oder aufgewiesen haben. Hierbei kann zumindest ein weiteres personenbezogenes Datum vom ersten Benutzer B1 und in den Datensätzen DSi angegeben werden, das einen Zeitraum seit dem erstmaligen Auftreten der Krankheit oder des Symptoms oder Zeitraum seit dem Abklingen der Krankheit oder des Symptoms angibt. In diesen Verfahren kann insbesondere der Schweregrad eines Symptoms bestimmt werden, wobei die Schwere des Symptoms das Leistungsergebnis des Leistungsmesswerts des ersten Benutzers innerhalb der herangezogenen Untergruppe ist. Anhand dieses Ergebnisses kann eine Physiotherapie oder Rehabilitation ausgerichtet werden.

Das beschriebene Verfahren könnte jedoch auch im Sport, insbesondere Leistungssport, eingesetzt werden, um den Leistungsgrad eines Sportlers zu bestimmen. Hierbei umfasst die Datenbank zumindest zwei, bevorzugt über hundert, Datensätze von Sportlern, die im selben Sport wie der erste Benutzer B1 tätig sind. Anhand des Leistungsergebnisses, das den Leistungsgrad des ersten Benutzers angibt, kann ein Trainingsplan veranschlagt werden.

## Patentansprüche

1. Computerimplementiertes Verfahren zur Durchführung einer Leistungsanalyse eines ersten Benutzers (B1), umfassend die Schritte:
- Bereitstellen einer Datenbank (1) mit einer Vielzahl von Datensätzen (DSi), wobei jeder Datensatz (DSi) eine Vielzahl von personenbezogenen Daten (pD) und zumindest einen ersten Leistungsmesswert (LM) umfasst,
- Empfangen von personenbezogenen Daten (pDB1-1, pDB1-2) des ersten Benutzers (B1) und eines gemessenen Leistungsmesswertes (LMB1-1) des ersten Benutzers (B1) oder einer Indikation eines zu messenden Leistungsmesswertes (LMB1-1) des ersten Benutzers (B1),
- Bestimmen von zumindest zwei unterschiedlichen Untergruppen (UG1, UG2, UG3) der Datenbank (1), wobei jede Untergruppe (UG1, UG2, UG3) nur jene Datensätze (DSi) der Datenbank (1) umfasst, bei der ein ausgewähltes Datum oder eine ausgewählte Kombination von zumindest zwei Daten der personenbezogenen Daten (pD) mit einem entsprechenden Datum oder einer entsprechenden Kombination der personenbezogenen Daten (pDB1-1, pDB1-2) des ersten Benutzers (B1) übereinstimmt oder in Verbindung steht,
- für die genannten Untergruppen (UG1, UG2, UG3), Ermitteln zumindest einer statistischen Eigenschaft, insbesondere einer Varianz oder Standardabweichung, aus den Leistungsmesswerten (LM) der Untergruppen (UG1, UG2, UG3),
- Heranziehen jener Untergruppe (UG1, UG2, UG3), die der gemäß der zumindest einen ermittelten statistischen Eigenschaft am repräsentativsten für den ersten Benutzer (B1) ist, bevorzugt durch Durchführung einer Diskriminanzanalyse,
- Durchführen der Leistungsanalyse des ersten Benutzers (B1) durch Ermitteln eines Leistungsergebnisses des Leistungsmesswerts (LMB1-1) des ersten Benutzers (B1) innerhalb der herangezogenen Untergruppe (UG1, UG2, UG3).

2. Verfahren nach Anspruch 1, wobei im Schritt des Bestimmens von zumindest zwei unterschiedlichen Untergruppen (UG1, UG2, UG3) alle möglichen Untergruppen (UG1, UG2, UG3) bestimmt werden.

3. Verfahren nach Anspruch 1 oder 2, wobei eine statistische Eigenschaft nur dann für eine zuvor bestimmte Untergruppe (UG1, UG2, UG3) ermittelt, wenn die Untergruppe (UG1, UG2, UG3) statistisch relevant ist, insbesondere wenn die Untergruppe (UG1, UG2, UG3) eine vorbestimmte Anzahl von Datensätzen (DSi) umfasst.

4. Verfahren nach einem der vorherstehenden Ansprüche, wobei das Verfahren die folgenden Schritte umfasst:
- Empfangen eines gemessenen zweiten Leistungsmesswertes (LMB1-2) des ersten Benutzers (B1) oder Empfangen einer Indikation eines zu messenden zweiten Leistungsmesswertes (LMB1-2) des ersten Benutzers (B1),
- Wiederholen der Schritte Ermitteln, Heranziehen und Durchführen für den zweiten Leistungsmesswert (LMB1-2).

5. Verfahren nach Anspruch 4, wobei für die beiden Leistungsmesswerte (LMB1-1, LMB1-2) unterschiedliche Untergruppen (UG1, UG2, UG3) herangezogen werden, wobei sich die Untergruppen (UG1, UG2, UG3) besonders bevorzugt überlappen.

6. Verfahren nach einem der vorherstehenden Ansprüche, wobei eine weitere Untergruppe (UG1, UG2, UG3) herangezogen wird und eine weitere Leistungsanalyse des ersten Benutzers (B1) durch Ermitteln eines weiteren Leistungsergebnisses des Leistungsmesswerts (LMB1-1) innerhalb der weiteren herangezogenen Untergruppe (UG1, UG2, UG3) durchgeführt wird, wobei beide Leistungsergebnisse bevorzugt als vergleichende Darstellung auf einem Bildschirm angezeigt werden.

7. Verfahren nach einem der vorherstehenden Ansprüche, wobei die Schritte Empfangen, Bestimmen, Ermitteln, Heranziehen und Durchführen für einen zweiten Benutzer wiederholt werden, wobei der Typ des Leistungsmesswertes (LMB1-1) des ersten Benutzers (B1) dem Typ des Leistungsmesswertes (LMB1-1) des zweiten Benutzers entspricht, und wobei die herangezogenen Untergruppen (UG1, UG2, UG3) für den ersten Benutzer und den zweiten Benutzer bevorzugt unterschiedlich sind.

8. Verfahren nach Anspruch 7, wobei die Leistungsmesswerte des ersten Benutzers (B1) und des zweiten Benutzers im Wesentlichen zeitgleich ermittelt werden und die Leistungsergebnisse des ersten Benutzers (B1) und des zweiten Benutzers bevorzugt als vergleichende Darstellung auf einem Bildschirm angezeigt werden.

9. Verfahren nach einem der vorherstehenden Ansprüche, wobei die personenbezogenen Daten (pDB1-1, pDB1-2) und die gemessenen Leistungsmesswerte (LMB1-1) des ersten Benutzers (B1) der Datenbank (1) hinzugefügt werden, nachdem die Leistungsanalyse durchgeführt wurde.

10. Verfahren nach einem der vorherstehenden Ansprüche, wobei für die genannten Untergruppen (UG1, UG2, UG3), für die zumindest eine statistische Eigenschaft ermittelt wurde, auf Basis der ermittelten statistischen Eigenschaften ein Repräsentationsfaktor ermittelt wird, wobei die Untergruppe mit dem größten Repräsentationsfaktor als repräsentativste Untergruppe bestimmt wird, wobei bevorzugt zumindest ein Repräsentationsfaktor oder zumindest zwei Repräsentationsfaktoren von zumindest zwei Untergruppen ausgegeben und besonders bevorzugt auf einem Bildschirm angezeigt werden.

11. Verfahren nach einem der vorherstehenden Ansprüche, wobei zumindest eine der ermittelten statistischen Eigenschaften eine Varianz oder Standardabweichung der Leistungsmesswerte der Untergruppen ist und eine Untergruppe umso repräsentativer ist, desto geringer ihre Varianz oder Standardabweichung ist.

12. Verfahren nach einem der vorherstehenden Ansprüche, wobei zumindest eine der ermittelten statistischen Eigenschaften vom Leistungsmesswert des ersten Benutzers (B1) abhängig ist,
wobei die genannte statistische Eigenschaft bevorzugt der Abstand des Leistungsmesswert des ersten Benutzers zum Mittelwert der Leistungsmesswerte der Untergruppe und eine Untergruppe umso repräsentativer ist, desto geringer der Abstand des Leistungsmesswert des ersten Benutzers (B1) zum Mittelwert der Leistungsmesswerte der Untergruppe ist.

13. Verfahren nach einem der vorherstehenden Ansprüche zur Bestimmung des Schweregrads eines Symptoms, wobei die Schwere des Symptoms das Leistungsergebnis des Leistungsmesswerts (LMB1-1) des ersten Benutzers (B1) innerhalb der herangezogenen Untergruppe (UG1, UG2, UG3) ist.

14. Verfahren nach einem der vorherstehenden Ansprüche zur Bestimmung eines Leistungsgrades eines Sportlers, wobei der Leistungsgrad das Leistungsergebnis des Leistungsmesswerts (LMB1-1) des ersten Benutzers (B1) innerhalb der herangezogenen Untergruppe (UG1, UG2, UG3) ist.

15. Vorrichtung zur Datenverarbeitung, die dazu ausgebildet ist, das Verfahren gemäß einem der Ansprüche 1 bis 14 durchzuführen.
